Europäisches Patentamt

European Patent Office

Office européen des brevets

(11) Publication number: **0 247 142 B1**

(12) **EUROPEAN PATENT SPECIFICATION**

(45) Date of publication of patent specification: **07.01.93**

(51) Int. Cl.5: **A61K 7/043**, A61K 31/78, A61K 31/44

(21) Application number: **86907064.9**

(22) Date of filing: **31.10.86**

(86) International application number:
**PCT/US86/02292**

(87) International publication number:
**WO 87/02580 (07.05.87 87/10)**

(54) **FILM-FORMING, PHARMACEUTICAL VEHICLES FOR APPLICATION OF MEDICAMENTS TO NAILS, PHARMACEUTICAL COMPOSITIONS BASED ON THOSE VEHICLES, AND METHODS OF USING SAME.**

(30) Priority: **04.11.85 US 794361**

(43) Date of publication of application:
**02.12.87 Bulletin  87/49**

(45) Publication of the grant of the patent:
**07.01.93 Bulletin  93/01**

(84) Designated Contracting States:
**AT BE CH DE FR GB IT LI LU NL SE**

(56) References cited:
**EP-A- 5 857          FR-A- 2 060 406
GB-A- 2 085 297      JP-A-59 216 822
US-A- 2 799 613      US-A- 2 972 545
US-A- 3 749 769**

**G.A. NOWAK: "Kosmetischen Präparate" Rezeptur, Rohstoffe, Wissenschaftliche Grundlagen, vol. 2, 3rd ed., 1984, Ziolkowsky Verlag, Augsburg, DE**

(73) Proprietor: **Owen/Galderma Laboratories Inc.
6201 South Freeway
Fort Worth, TX 76134(US)**

(72) Inventor: **HEBBORN, Peter
5500 Grissom Drive
ARLINGTON, TX 76016(US)**
Inventor: **ACHARYA, Ramesh, N.
7117 Bettis Drive
Fort Worth, TX 76133(US)**
Inventor: **BIDGOOD, Alison
1140 Woodvale Drive
Bedford, TX 76021(US)**
Inventor: **Patel, Dinesh, C.
5839 S. Meadow Crest Drive
Murray, UT 84107(US)**

(74) Representative: **West, Alan Harry et al
R.G.C. Jenkins & Co. 26 Caxton Street
London SW1H 0RJ(GB)**

EP 0 247 142 B1

Rank Xerox (UK) Business Services

Chemical Abstracts, vol. 94, 1981 (Columbus, Ohio, US), J.D. Nelson et al., "Sodium and Zinc Omadine Antimicrobials as Cosmetic Preservatives", see page 375, column 1, abstract no. 162626b, Cosmet. Toiletries, 1981, 96(3), 87-90 (Eng)

JUE et al,"Ciclopirox Olamine 1% Cream, A Prelimininary Review of its Antimicrobial Activity and Therapeutic Use" Drugs, Volume 29, issued 1985

**Description**

The present invention relates to film-forming vehicles for delivering medicaments to the nails of mammals. More particularly, this invention relates to a pharmaceutical composition comprising a film-forming vehicle which is adapted to deliver one or more active ingredients to human nails in the treatment of various onychopathic conditions, such as onychomycosis. These compositions provide a means for topical treatment of onychopathic conditions that is both effective and convenient.

Onychopathic conditions are very difficult to treat topically due to the poor delivery of drugs through the nails when the drugs are applied using conventional dosage forms, such as, creams, lotions, gels, solutions, and so on. This poor delivery is primarily attributable to the fact that the drugs are easily rubbed off, washed away or otherwise removed from the nails is when applied via the conventional dosage forms mentioned above, and as a result may only be in contact with the nails for a very limited time.

Human nails are typically 100 to 200 times thicker than the stratum corneum. Because of this greater thickness, the time required for a given quantity of drug to penetrate the nail is much greater than the time required for the same quantity of drug to penetrate the stratum corneum. Thus, the drug must be in contact with the nail for a relatively long time (e.g., several hours) in order to achieve a therapeutic concentration of drug in the affected tissue beneath the nail (i.e., the nail bed). The above-cited conventional dosage forms are generally ineffective because the drug is not kept in contact with the nail long enough to achieve a therapeutic concentration of drug in the nail bed.

The prior art approaches to treating onychopathic conditions have generally fallen into one of three categories. Namely, these approaches have relied on either systemic (e.g., oral) administration of drugs; surgical removal of all or part of the nail followed by topical application of drugs to the thus exposed tissue beneath the nail; or topical application of conventional crews, lotions, gels or solutions, frequently including the use of bandages to keep these dosage forms in place on the nails. All of these prior art approaches suffer from major drawbacks.

A major problem seen with systemic therapy is the considerable amount of time which is usually required to produce a therapeutic effect in the nail bed. For example, oral treatment of onychomycoses with the antifungal compound ketoconazole typically requires administration of a 200 to 400 mg daily dose for an average period of 20-25 weeks before any significant therapeutic effect is realized. The potential for side effects on major organ systems is a very significant danger associated with such long term, systemic therapy, since ketoconazole has been reported to produce fatalities attributable to liver toxicity and to reduce testosterone levels in the blood due to an adverse effect on the testes. A further drawback seen with this type of therapy is the greater expense to the patient and consequent adverse affect on patient compliance.

A major drawback seen with the second type of therapy mentioned above, namely removing all or part of the nail prior to topical treatment of the condition, is the discomfort experienced by the patient, both during and subsequent to removal of the nail. A further problem with this approach is the undesirable cosmetic appearance of the nail or nail bed; this may represent a significant problem for some patients, particularly female patients.

A major problem seen with the third prior art approach to therapy has already been mentioned above; namely, conventional dosage forms such as creams, lotions, gels or solutions are easily removed from the nails. The use of bandages or similar means in an attempt to eliminate this problem has generally been met with poor patient compliance. The poor patient compliance is understandable, since such bandages are typically awkward and become very troublesome to keep in place over extended periods of several weeks or more. The use of a 2% miconazole cream together with occlusive bandages to treat onychomycosis is discussed in the following article: E. Heinke, "Clinical experiences with miconazol with special regard to a conservative treatment of the onychomycoses and paronychia", Mykosen, Vol. 15, pp. 405-407 (1972).

A 2% miconazole alcoholic solution is described in the following article: J. Vanderdonckt et al., "Miconazole alcoholic solution in the treatment of mycotic nail infections", Mykosen, Vol. 19, pp. 251-256 (1976). The solution is said to have the following formula:

| | |
|---|---|
| Miconazole base | 0.2 gram |
| Carbosept No. 525 | 0.475 gram |
| Carbosept No. 515 | 0.025 gram |
| Propylene glycol USP | 2 gram |
| Ethanol USP | ad 10 mL |

EP 0 247 142 B1

The article characterizes the above-identified solution as being a "film-forming alcoholic solution." (Emphasis added.) However, experiments conducted by the present inventors have shown that in fact the vehicle utilized by Vanderdonckt et al., (i.e., all of the ingredients listed above except for the miconazole) does not produce a continuous, self-supporting film, but rather leaves a deposit on the nails which may be more readily described as a residue than as a film. For purposes of the present specification, a film is considered to be self-supporting when it can be peeled from a nonstick surface such as glass or Teflon®. That is, a film is considered to be self-supporting when it can be removed from a nonstick surface and still retain the physical characteristics and form of a continuous film.

The experiments conducted by the present inventors have shown that the residue formed by the above-identified solution takes several hours to dry and disintegrates if placed in water. In contrast, the film-forming vehicles of the present invention generally require only a few minutes to dry when placed on human nails and do not disintegrate readily when contacted with water. These properties of the film-forming vehicles of the present invention are very significant with respect to the patient compliance and efficacy seen with compositions based on these vehicles.

The above-cited article also states that the solution was applied to the nails in drop form twice daily. This is a further indication that the alcoholic solution utilized by Vanderdonckt et al., is significantly different from the film-forming vehicles of the present invention, which form a continuous film, adhere readily to the nail, and may be applied as infrequently as once or twice per week. Additional reports on the use of the above-identified 2% miconazole alcoholic solution are set forth in the following articles: G. Achten et al., "Treatment of onychomycosis with a solution of miconazole 2% in alcohol," Mykosen, Vol. 20, pp. 251-256 (1976); and B. Bentley-Phillips, "The treatment of onychomycosis with miconazole tincture," South African Medical Journal, Vol. 62, pp. 57-58 (1982).

Japanese Patent Application No. 58-92926 (preliminary publication No. 59-216822) discloses film-forming, endermic formulations. Those formulations differ from the film-forming vehicles of the present invention in that, among other things, the films formed are specially adapted to be flexible so as to remain on the skin after drying. A considerable amount (i.e., 5 to 1500 parts by weight) of a plasticizer (e.g., propylene glycol) is included in the formulations in order to provide this flexibility. It is believed that these formulations would require a considerable amount of time to dry if painted on human nails due to the relatively large amount of plasticizer contained therein. The film-forming vehicles of the present invention are specially adapted for use on nails rather than skin, and do not require the presence of a plasticizer in order to provide flexibility. Thus, the film-forming vehicles of the present invention are designed to form films which are relatively inflexible compared to those of the type described in the Japanese Application.

GB 2085297 describes a composition for the treatment of psoriasis of nails by topical application of a corticosteroid incorporated into a commercially available nail polish; there is no specific disclosure of the nail polish.

US 2799613 describes the fungicidal and bactericidal properties of dibromo-pentachlorocyclohexane and mentions briefly that such a compound can be incorporated into cosmetics including nail polish. However, the molecular weight and probable water solubility of dibromo-pentachloro-cyclohexane clearly indicates that it is unsuitable for the topical treatment of fungal nail infections.

The foregoing discussion demonstrates that a need exists for an effective, topical regimen for treating onychopathic conditions. More specifically, a need exists for an effective means of applying drugs topically to the nails in a manner which will, among other things, bring about:

(1) prolonged presence of drug on the nails;

(2) relatively rapid therapeutic concentrations of drug in the nail bed; and

(3) improved patient compliance based on convenience and cosmetic appearance.

A principal object of the invention is to provide a pharmaceutical composition for use in the treatment of fungal nail infections comprising a film-forming vehicle which is adapted to deliver drugs to nails in an efficacious and cosmetically acceptable manner and to significantly enhance the delivery of drugs through nails by maintaining the drugs in contact with the nails.

Accordingly, the present invention provides an aqueous pharmaceutical composition for use in the treatment of fungal nail infections comprising:

a) a pharmaceutical vehicle comprising from 2% to 40% by weight of a hydrophilic, film-forming resin, and a pharmaceutically acceptable solvent for the resin, the vehicle being capable of forming a continuous, self-supporting, dry film following application to human nails;

b) a therapeutic amount of at least one antimycotic drug selected from sodium propionate, sodium pyrithione, ciclopirox and pharmaceutically acceptable salts thereof; and

c) water.

The film-forming aqueous compositions of the present invention are based on hydrophilic, filmforming resins. The film-forming resins utilized are polymeric materials which are at least partially soluble in water and/or polar organic solvents (e.g. methanol, ethanol, isopropanol, acetone, chloroform, methylene chloride, and the like), and which are capable of producing a continuous, self-supporting film upon evaporation of the solvent. As mentioned above, the films formed by the vehicles of the present invention are considered to be self-supporting because it is possible to remove the films from a nonstick surface and still retain the physical characteristics and form of a continuous film. The film-forming resins are hydrophilic in the sense that they have an affinity for water or polar organic solvents, and are wettable by water. Examples of such film-forming resins include: cellulose resins, ethylene oxide resins, acrylic acid and acrylate resins, vinyl resins and vinyl pyrrolidone resins.

Examples of suitable cellulose resins include: methyl cellulose, carboxymethyl cellulose, hydroxypropylcellulose, hydroxypropylmethyl cellulose and hydroxyethylcellulose.

Examples of suitable ethylene oxide resins include polyethylene oxide polymers.

Examples of suitable acrylic acid and acrylate resins include: polyacrylamide, polyacrylic/polymethacrylic, polymethacrylate/butylacrylate, polyacrylic/acrylate and polyacrylic acid polymer.

Examples of suitable vinyl resins include: polyvinyl alcohol and poly (methyl vinyl ether/maleic anhydride).

Examples of suitable vinyl pyrrolidone resins include: polyvinyl pyrrolidone, polyvinyl pyrrolidone/vinyl acetate, and vinyl pyrrolidone/dimethylaminethyl methacrylate.

The above-described hydrophilic, film-forming resins are commercially available. The resins which may be utilized in the present invention are further illustrated by the specific examples set forth in Table 1 below.

Table 1

| Class | Subclass | Tradename | Manufacturer | Molecular Weight Range |
|---|---|---|---|---|
| Cellulose Resins | Alkyl Cellulose | Methocel A | Dow Chemical | 50,000 - 1,000,000 |
| | Hydroxyakyl Alkyl Cellulose | Methocel E | Dow Chemical | |
| | Carboxymethyl Cellulose | CMC | Industries of India | 50,000 - 700,000 |
| | | CMC-T | Hercules Chemicals | |
| | Hydroxy Alkyl Cellulose | HEC HPC Klucel Natrosol | Hercules Chemicals | 50,000 - 1,200,000 |
| Ethylene Oxide Resins | Polyethylene Oxide Polymers | Polyox WSR N-10 Polyox WSR N-80 Polyox WSR N-750 Polyox WSR N-3000 Polyox WSR 205 Polyox WSR 1105 Polyox WSR 301 | Union Carbide | 50,000 - 4,000,000 |

EP 0 247 142 B1

Table 1 - Continued

| Class | Subclass | Tradename | Manufacturer | Molecular Weight Range |
|---|---|---|---|---|
| Acrylic Acid and Acrylate Resins | Poly (acrylic acid) | *Acrysol-1<br>*Acrysol-3<br>*Acrysol-5 | Rohm and Haas | 50,000 – 400,000 |
| | | Carbopol 910<br>Carbopol 934<br>Carbopol 940<br>Carbopol 941 | B. F. Goodrich | 400,000 – 5,000,000 |
| | Polyacrylamide | *Poly Trap 158<br>*Poly Trap FML 205 | Wickhen Chemicals | 10,000 – 1,000,000 |
| | Acrylyamide/ Sodium Acrylate | Reten-421<br>Reten-423<br>Reten-425 | Hercules Chemicals | 10,000 – 1,000,000 |
| | Acrylic/Acrylate Copolymer | Carboset-514<br>Carboset-515<br>Carboset-525 | B. F. Goodrich | 7,000 – 1,000,000 |
| | Polymethacrylate/ Butyl Acrylate Copolymer | Plastoid A<br>Plastoid B | Rohm Pharm. Co. | 25,000 – 1,000,000 |
| Vinyl Resins | Polyvinyl Alcohol | Gohsenol<br>Gelvatol<br>Elvanol | Nippon Gohesie<br>Monsanto<br>E. I. DuPont | 25,000 – 300,000 |

Continued . . .

EP 0 247 142 B1

Table 1 - Continued

| Class | Subclass | Tradename | Manufacturer | Molecular Weight Range |
|---|---|---|---|---|
| Polyvinyl Pyrrolidone Resins | Polyvinyl Pyrrolidone Polymers | PVP K-25<br>PVP K-30<br>PVP K-90 | G. A. F. Chemicals | 15,000 - 800,000 |
| | Polyvinyl Pyrrolidone/Vinyl Acetate Copolymers | **PVP/VA | G. A. F. Chemicals | 50,000 - 1,000,000 |
| | Polyvinyl Pyrrolidone/Dimethylamino-ethyl Methacrylate Copolymers | GAFQUAT | G. A. F. Chemicals | 100,000 - 1,000,000 |

*These products are actually formulations containing the indicated type of resin rather than pure resin.

**A series of polyvinyl pyrrolidone/vinyl acetate copolymers containing the following ratios of PVP/VA: 70/30, 60/40, 50/50, 30/70, and 20/80.

The above-identified film-forming, polymeric resins are further described in the following publications: Handbook of Water Soluble Gums and Resins, Davidson, R. L., McGraw-Hill Book Company (1980); and "Gum Technology in the Food Sciences," Food Science and Technology - A Series of Monographs, Glicksman, M., Academic Press (1969). The entire contents of these publications are incorporated herein by reference.

One or more of the above-described, hydrophilic film-forming resins are contained in the film-forming vehicles of the compositions according to the invention in an amount of from about 2% to about 40% by weight. The amount of polymeric resin utilized in specific vehicles will vary depending on factors such as the molecular weight, viscosity and solubility of the resin; the type of solvent utilized; the inclusion of other components in the vehicles; and the type of active ingredient contained in the vehicles. The amount of polymeric resin contained in the vehicles will also depend to some extent on the particular type of resin utilized. The preferred ranges for each of the five classes of resins identified in Table 1 are as follows:

| Class | Preferred Range (wt %) |
|---|---|
| Cellulose Resins | 5-15 |
| Polyethylene Oxide Resins | 10-30 |
| Acrylic Acid and Acrylate Resins | 5-35 |
| Vinyl Resins | 10-30 |
| Polyvinyl Pyrrolidone | 10-30 |

The film-forming vehicles described above are optionally used in combination with a layer of polymeric material which is applied to the nail after the film-forming vehicle has dried. The use of this second layer of material in effect creates a two coat system on the nail, with the first coat comprising the film-forming vehicle and drug and the second coat comprising the layer of polymeric material or overcoat.

The two coat system generally enhances the ability of drugs to penetrate the nail by modifying the hydration characteristics of the nails and the film-forming vehicles.

The two coat system is also advantageous in that it generally allows a greater concentration of drug to be contained in the film-forming vehicles. More particularly, when higher concentrations of drug are contained in the film-forming vehicles, the vehicles exhibit a greater tendency to deteriorate when submerged in water. The overcoat layer protects the film-forming vehicles from such deterioration, and thereby enables a greater concentration of drug to be contained in the vehicles.

The overcoat layer should preferably be waterproof, and must be capable of drying quickly (i.e., within 10 to 15 minutes, preferably within 1-2 minutes). Various types of materials meeting these requirements may be utilized. For example, most commercially available nail lacquers and polishes can be utilized for this purpose. This is a distinct advantage in the treatment of many female patients, since the ability to use nail polishes for the overcoat layer in the two coat system may further contribute to good patient compliance by allowing the patient to maintain the normal cosmetic appearance of the nails while undergoing treatment.

Although commercially available nail lacquers and polishes may be utilized for the overcoat layer, the film-forming vehicles of the present invention and solutions containing pyroxylin resin (e.g., collodion USP and flexible collodion USP) may also be utilized for this purpose.

The pharmaceutical compositions of the present invention may further comprise one or more chemical agents to modify the physical and/or chemical properties of the vehicles and films formed from the vehicles. Examples of physical and chemical properties of the vehicles which may be modified include: solubility of the film-forming resins and drugs, viscosity, suspendability, clarity, light and UV absorption, and drying. Examples of the physical properties of films which may be modified include: elasticity, hardness, brittleness, appearance, adherence, drying and tackiness.

The classes of chemical agents which may be utilized to modify the above-described properties include: humidity controlling agents and tack controlling agents. Examples of suitable tack control agents include: carboxymethyl cellulose, cellulose, cellulose acetate proprionate, colloidal silica and shellac. Examples of suitable humidity controlling agents include glycerin and sorbitol.

The specific chemical agents utilized to modify the above-discussed properties and the amount of such agents required are dependent on numerous factors, but are primarily dependent on the desired drying time of the film. In general, it is desired that the film-forming vehicles of this invention be capable of forming a dry film within 15 minutes following application to the nails, preferably within 5 minutes. The films are deemed "dry" when tack free if touched lightly with a finger.

In addition to the above-described components, the compositions of the invention may also include various adjuvants conventionally utilized in topically applied pharmaceutical dosage forms, such as, antioxidants, antimicrobial preservatives, chelating agents, buffering agents, pigments, ultra-violet absorbers, and so on.

The film-forming vehicles described are used to deliver drugs to the nails. Because of the hydrophilic properties of the resins, films formed from the resins support the diffusion of drugs contained in the film matrix. The drugs which may be delivered must be physically and chemically compatible with the film-forming vehicle and be capable of permeating the nail. With regard to the latter requirement, it has been determined that drugs having a molecular weight of greater than about 550 can not effectively permeate the nail. Accordingly, the drugs which may be utilized in the compositions of the present invention must have a molecular weight less than about 550, and preferably have a molecular weight less than about 300. It has also been determined that drugs having a water solubility of greater than about 0.01 percent by weight generally permeate the nail more readily than drugs having a lesser degree of water solubility. Drugs having a water solubility of greater than about 0.01 percent by weight are therefore preferred in the present invention.

A particularly important classy of drug which are useful in the treatment of onychopathic conditions is the antimycotics since fungal infections of nails and nail beds are typically very difficult to treat using conventional prior art therapies. The film-forming vehicles of the present invention are particularly useful for delivering drugs having antimycotic activity to the nails. Specifically the drugs used in the compositions according to the present invention are, ciclopirox, sodium propionate, sodium pyrithione, and pharmaceutically acceptable salts thereof. These drugs are all known compounds which are further described, for example, in The Merck Index, Tenth Edition (1983).

The amount of the above drugs contained in the compositions of the present invention will vary widely depending on the severity of the condition, and other factors. In general, the compositions will contain from about 0.01% to about 25% by weight at least one of the antimycotic drugs listed above.

The present invention is further illustrated by the following examples.

Examples 1

All percentages are by weight based on the total weight of the composition.

| Ingredient | % |
|---|---|
| Carboset 525 | 18 |
| Omadine (sodium pyrithione) | 1 |
| Purified Water | 15 |
| Alcohol (SD 40-2) | QS 100 |

This composition was prepared by combining all of the ingredients except for the Carboset 525 resin to form a mixture, and then adding the Carboset 525 resin to that mixture. The resulting mixture was stirred with a paddle stirrer until the Carboset 525 resin was dissolved. The resulting product was a clear solution having a straw color. When painted on nails as a thin layer, this product provided a tack-free, transparent film within approximately three minutes following application.

Example 2

All percentages are by weight based on the total weight of the composition.

| Ingredient | % |
|---|---|
| Polyvinyl Alcohol (Vinyl Resin) | 25.0 |
| Sodium Propionate (Antifungal) | 1.0 |
| Water | QS 100 |

This composition may be prepared by dissolving the polyvinyl alcohol in water with stirring and dissolving the sodium propionate in the resulting solution.

Example 3

The following formulation illustrates a composition which is suitable for use as the overcoat layer. All percentages are by weight based on the total weight of the composition.

| Ingredient | % |
|---|---|
| Camphor | 2 |
| Castor Oil | 3 |
| Collodion USP | QS 100 |

The ingredients are combined and shaken to form a solution.

Example 4

The following experimental results demonstrate the efficacy of the present compositions in treating onychopathic conditions, specifically onychomycoses.

A fifty-two year old, white male patient, afflicted with an onychomycotic infection of the big toe, had treated the infection topically with applications of either Tinactin™ (tolnaftate) solution or Micatin™ - (miconazole) cream twice daily for many months without producing any clinical signs of improvement. This patient was treated with a composition in accordance with the present invention containing 5.0 wt.% sodium omadine (sodium pyrithione) and based on the vehicle illustrated in Example 1. The composition was applied to the affected toe by painting a single layer on the nail, and waiting approximately two minutes for the layer to form a dry, tack-free film. The composition was applied in this manner two times per week beginning in October 1984. By December 1984, obvious clinical improvement was apparent based on the growth of a normal appearing toe nail. By June 1985, the nail no longer exhibited any clinical signs of fungal infection.

## Claims

1.  An aqueous pharmaceutical composition for use in the treatment of fungal nail infections comprising:
    a) a pharmaceutical vehicle comprising from 2% to 40% by weight of a hydrophilic, film-forming resin, and a pharmaceutically acceptable solvent for the resin, the vehicle being capable of forming a continuous, self-supporting, dry film following application to human nails;
    b) a therapeutic amount of at least one antimycotic drug selected from sodium propionate, sodium pyrithione, ciclopirox and pharmaceutically acceptable salts thereof; and
    c) water.

2.  A pharmaceutical composition according to claim 1, in which the film-forming resin is selected from cellulose resins, ethylene oxide resins, acrylic acid and acrylate resins, vinyl resins, and polyvinyl pyrrolidone resins; and the vehicle is capable of forming a dry film within 15 minutes following application to human nails.

3.  A pharmaceutical composition according to claim 2, in which the film-forming resin is acrylic acid resins or acrylate resins.

4.  A pharmaceutical composition according to any preceding claim, in which the pharmaceutical vehicle comprises 5 to 35% by weight of a hydrophilic film forming resin.

5.  A pharmaceutical composition according to any preceding claim, which forms a film over the affected nail thereby permitting the drug to permeate the nail and which comprises also a polymeric material which forms an overcoat layer.

6.  A pharmaceutical composition according to any preceding claim in which the antimycotic drug is sodium pyrithione.

7.  A pharmaceutical composition according to any preceding claim in which the pharmaceutical vehicle comprises from 5% to 30% by weight of a hydrophilic, film-forming resin, and wherein said drug comprises an antimycotic effective amount of sodium pyrithione.

## Patentansprüche

1.  Wässrige pharmazeutische Zusammensetzung zur Verwendung in der Behandlung von Nagel-Pilzinfek-

11

EP 0 247 142 B1

tionen, umfassend:

a) ein pharmazeutisches Vehikulum, welches 2 bis 40 Gewichts-% eines hydrophilen, filmbildenden Harzes und ein pharmazeutisch annehmbares Lösungsmittel für das Harz enthält, wobei das Vehikulum imstande ist, einen kontinuierlichen, selbstragenden, trockenen Film im Anschluß an die Applikation auf menschliche Nägel zu bilden;

b) eine therapeutische Menge von mindestens einem antimykotischen Arzneimittel, welches aus Natriumpropionat, Natriumpyrithion, Cyclopirox und deren pharamzeutisch annehmbaren Salzen ausgewählt ist; und

c) Wasser.

**2.** Pharmazeutische Zusammensetzungen nach Anspruch 1, **dadurch gekennzeichnet,** daß der filmbildende Harz ausgewählt ist aus Celluloseharzen, Ethylenoxidharzen, Acrylsäure- und Acrylatharzen, Vinylharzen und Polyvinylpyrrolidonharzen, und daß das Vehikulum imstande ist, einen trockenen Film innerhalb von 15 Minuten im Anschluß an die Applikation auf menschliche Nägel zu bilden.

**3.** Pharmazeutische Zusammensetzung nach Anspruch 2, **dadurch gekennzeichnet,** daß das filmbildende Harz Acrylsäureharz oder Acrylatharz ist.

**4.** Pharmazeutische Zusammensetzung nach einem der vorhergehenden Ansprüche, **dadurch gekennzeichnet,** daß das pharmazeutische Vehikulum 5 bis 35 Gewichts-% eines hydrophilen, filmbildenden Harzes enthält.

**5.** Pharmazeutische Zusammensetzung nach einem der vorhergehenden Ansprüche, **dadurch gekennzeichnet,** daß sie über dem befallenen Nagel einen Film bildet, wodurch dem Arzneimittel gestattet wird, den Nagel zu durchdringen, und die ebenso ein Polymermaterial umfaßt, welches eine Überzugsschicht bildet.

**6.** Pharmazeutische Zusammensetzung nach einem der vorhergehenden Ansprüche, **dadurch gekennzeichnet,** daß das antimykotische Arzneimittel Natriumpyrithion ist.

**7.** Pharmazeutische Zusammensetzung nach einem der vorhergehenden Ansprüche, **dadurch gekennzeichnet,** daß das pharmazeutische Vehikulum 5 bis 30 Gewichts-% eines hydrophilen, filmbildenden Harzes enthält, und daß das Arzneimittel eine antimykotisch wirksame Menge an Natriumpyrithion umfaßt.

**Revendications**

**1.** Composition pharmaceutique aqueuse destinée à être utilisée dans le traitement des infections fongiques des ongles, comprenant :

(a) un véhicule pharmaceutique comprenant de 2% à 40% en poids d'une résine filmogène, hydrophile, et un solvant pharmaceutiquement acceptable pour la résine, le véhicule étant capable de former un film sec, autoporteur, continu, après application sur les ongles humains

(b) une quantité thérapeutique d'au moins un médicament antimycotique choisi parmi le propionate de sodium, la pyrithione sodique, le ciclopirox et leurs sels pharmaceutiquement acceptables ; et

(c) de l'eau.

**2.** Composition pharmaceutique selon la revendication 1, dans laquelle la résine filmogène est choisie parmi les résines de cellulose, les résines d'oxyde d'éthylène, les résines d'acide acrylique et d'acrylate, les résines vinyliques et les résines de polyvinyl pyrrolidone ; et le véhicule est capable de former un film sec en l'espace de 15 minutes après application sur les ongles humains.

**3.** Composition pharmaceutique selon la revendication 2, dans laquelle la résine filmogène est choisie parmi les résines d'acide acrylique et les résines d'acrylate.

**4.** Composition pharmaceutique selon l'une quelconque des revendications précédentes, dans laquelle le véhicule pharmaceutique comprend 5 à 35% en poids d'une résine filmogène hydrophile.

**5.** Composition pharmaceutique selon l'une quelconque des revendications précédentes, qui forme un film sur l'ongle affecté, permettant ainsi au médicament de pénétrer l'ongle, et qui comprend également une matière polymère qui forme une couche de revêtement supérieur.

6. Composition pharmaceutique selon l'une quelconque des revendications précédentes, dans laquelle le médicament antimycotique est la pyrithione sodique.

7. Composition pharmaceutique selon l'une quelconque des revendications précédentes, dans laquelle le véhicule pharmaceutique comprend de 5% à 30% en poids d'une résine filmogène, hydrophile, et dans laquelle ledit médicament comprend une quantité antimycotique efficace de pyrithione sodique.z